# EUROPEAN PATENT SPECIFICATION

(11) **EP 1 660 066 B1**
(45) Date of publication and mention of the grant of the patent: **28.10.2009**
(21) Application number: 04764321.8
(22) Date of filing: 20.08.2004
(51) Int. Cl.: A61K 31/155, A61K 31/366, A61K 31/404, A61K 31/40, A61K 31/506, A61P 3/06, A61P 3/10, A61P 9/00

(54) **PHARMACEUTICAL COMPOSITION COMPRISING A COMBINATION OF METFORMIN AND A STATIN**
PHARMAZEUTISCHE ZUSAMMENSETZUNG MIT EINER KOMBINATION AUS METFORMIN UND EINEM STATIN
COMPOSITION PHARMACEUTIQUE COMPRENANT UNE COMBINAISON DE METFORMINE ET UNE STATINE

(30) Priority: 22.08.2003 EP 03292077
(43) Date of publication of application: 31.05.2006
(73) Proprietor: Fournier Laboratories Ireland Limited, Carrigtwohill, Co. Cork (IE)
(72) Inventor: JUNIEN, Jean-Louis, F-92310 SEVRES (FR); EDGAR, Alan, F-21490 SAINT JULIEN (FR)
(74) Representative: Hart-Davis, Jason
(86) International application number: PCT/EP2004/009337
(87) International publication number: WO 2005/018626

(56) References cited:
- ZUNZUNEGUI-PASTOR M V ET AL: "EFFECTS OF CANDESARTAN, METFORMIN AND PRAVASTATIN COMBINATION ON CARDIOVASCULAR RISK FACTORS IN A POOR CONTROLED HYPERTENSIVE POPULATION WITH CHRONIC METABOLIC SYNDROME" AMERICAN JOURNAL OF HYPERTENSION, NEW YORK, NY, US, vol. 14, no. 4, PART 2, April 2001 (2001-04), pages 113A-114A,ABP-253, XP001128773
- CARLSEN SVEN M ET AL: "Evidence for dissociation of insulin- and weight-reducing effects of metformin in non-diabetic male patients with coronary heart disease" DIABETES RESEARCH AND CLINICAL PRACTICE, vol. 39, no. 1, January 1998 (1998-01), pages 47-54, XP002269504 ISSN: 0168-8227
- CARLSEN SVEN M ET AL: "Metformin increases circulating tumour necrosis factor-alpha levels in non-obese non-diabetic patients with coronary heart disease" CYTOKINE, vol. 10, no. 1, January 1998 (1998-01), pages 66-69, XP002269505 ISSN: 1043-4666
- ALSHEIKH-ALI ALAWI A ET AL: "Risk of adverse events with concomitant use of atorvastatin or simvastatin and glucose-lowering drugs (Thiazolidinediones, Metformin, Sulfonylurea, Insulin, and Acarbose)" AMERICAN JOURNAL OF CARDIOLOGY, vol. 89, no. 11, 1 June 2002 (2002-06-01), pages 1308-1310, XP002269506 ISSN: 0002-9149

## Description

This invention relates to the use, in the manufacture of a composition for treatment of patients with hyperglycaemia, of a combination of metformin and of a hydroxymethylglutaryl coenzyme A (HMG-CoA) reductase inhibitor or statin, such as, for example, pravastatin, lovastatin, simvastatin, atorvastatin, cerivastatin or fluvastatin, formulated together to provide simultaneously a therapeutically effective amount of the hydroxymethylglutaryl coenzyme A reductase inhibitor and a therapeutically effective amount of the metformin taken into the blood of a patient in need of treatment. The compositions to be used according to this invention are useful for controlling or reducing hyperglycaemia associated with non-insulin-dependent diabetes.

In humans, cholesterol and triglycerides (TG) are part of lipoprotein complexes in the bloodstream, and can be separated via ultracentrifugation into high-density lipoprotein (HDL), intermediate-density lipoprotein (IDL), low-density lipoprotein (LDL), and very-low-density lipoprotein (VLDL) fractions. Cholesterol and triglycerides are synthesized in the liver; incorporated into VLDL, and released into the plasma. High levels of total cholesterol (total-C), LDL-C, and apolipoprotein B (apo-B, a component of a membrane complex for LDL-C) promote human atherosclerosis, and decreased levels of HDL-C and its transport complex, apolipoprotein A, are associated with the development of atherosclerosis. Cardiovascular morbidity and mortality in humans can vary directly with the level of total-C and LDL-C and inversely with the level of HDL-C.

Orally administered statins are hydroxymethylglutaryl coenzyme A (HMG-CoA) reductase inhibitors that are used in patients to lower low density lipoprotein (LDL) cholesterol. Complementary to this are orally administered fibrates which are used in patients to decrease lipoproteins rich in triglycerides, to increase high density lipoprotein (HDL), and to decrease atherogenic-dense LDL. Patients who take statins or fibrates are frequently on diets with low and variable fat content.

HMG-CoA reductase (3-hydroxy-3-methylglutaryl-coenzyme A) is the microsomal enzyme that catalyses the rate limiting reaction in cholesterol biosynthesis (Mevalonate). According to the present invention, a statin is an HMG-CoA reductase inhibitor that inhibits HMG-CoA reductase, and therefore inhibits or interferes with the synthesis of cholesterol. Inhibition of cholesterol synthesis can lead to a reduction in blood cholesterol levels.

A large number of naturally or synthetically obtained or synthetically modified compounds have been found to inhibit HMG-CoA reductase. These compounds form a category of agents useful for the practice of the present invention. Traditionally these agents have been used to treat individuals with hypercholesterolemia.

Metformin is mainly known for its anti-hyperglycaemic activity and is widely used in the treatment of non-insulin-dependent diabetes. In the case of insulin-dependent diabetes, metformin is also administered to the patient in combination with insulin.

Zunzunegui-Pastor et al., American Journal of Hypertension, 14(4), April 2001, pages 113A-114A discloses the effect of a candesartan, metformin and pravastatin combination on cardiovascular risk factors in a population with poorly controlled hypertension and chronic metabolic syndrome.

Surprisingly, the present inventors have discovered that the combination of metformin with a statin leads to a significant improvement of the hyperglycaemia in a diabetic patient suffering from non-insulin-dependent diabetes. More specifically, a synergistic effect has been obtained by combined administration of metformin and a statin.

Thus, the invention relates to the use of metformin and a statin as active ingredients, and one or more pharmaceutically acceptable excipients for the manufacture of a pharmaceutical composition for controlling or decreasing glycaemia in non insulin dependent diabetes subjects.

The pharmaceutical composition to be used is more particularly suitable for reducing hyperglycaemia in non-insulin-dependent diabetes patients and to improve insulin resistance. It can also be used in non-dyslipidaemic patients or in dyslipidaemic patients.

The composition to be used in the present invention includes statins that are commercially available, such as lovastatin and mevinolin disclosed in U.S Pat. No. 4,231,938, pravastatin and pravastatin sodium disclosed in U.S. Pat. No. 4,346,227, fluvastatin and fluvastatin sodium and XU 62-320 disclosed in EP 0 114 027 and U.S. Pat. No. 4,739,073, atorvastatin disclosed in U.S. Pat. No. 5,273,995, itavastatin also known as NK-104 disclosed in EP304063, mevastatin disclosed in U.S. Pat. No. 3,983,140, rosuvastatin, velostatin and synvinolin and simvastatin disclosed in U.S. Pat. Nos. 4,448,784 and 4,450,171, cerivastatin, pitivastatin and numerous others described in U.S. Pat. Nos. 5,622,985, 5,135,935, 5,356,896, 4,920,109, 5,286,895, 5,262,435, 5,260,332, 5,317,031, 5,283,256, 5,256,689, 5,182,298, 5,369,125, 5,302,604, 5,166,171, 5,202,327, 5,276,021, 5,196,440, 5,091,386, 5,091,378, 4,904,646, 5,385,932, 5,250,435, 5,132,312, 5,130,306, 5,116,870, 5,112,857, 5,102,911, 5,098,931, 5,081,136, 5,025,000, 5,021,453, 5,017,716, 5,001,144, 5,001,128, 4,997,837, 4,996,234, 4,994,494, 4,992,429, 4,970,231, 4,968,693, 4,963,538, 4,957,940, 4,950,675, 4,946,864, 4,946,860, 4,940,800, 4,940,727, 4,939,143, 4,929,620, 4,923,861, 4,906,657, 4,906,624, RE36,520, and U.S. Pat. No. 4,897,402. The present invention is not limited to these statins.

Lovastatin, an inactive lactone, is a white, nonhygroscopic crystalline powder isolated from a strain of Aspergillus terreus that is insoluble in water and sparingly soluble in ethanol, methanol, and acetonitrile. Lovastatin is hydrolysed after oral ingestion to the corresponding (beta)-hydroxyacid. This metabolite is an inhibitor of 3-hydroxy-3-methylglutaryl-coenzyme A (HMG-CoA) reductase. When formulated for oral administration as Mevacor TM, tablets can contain 10 to 40 mg of lovastatin together with pharmaceutically acceptable excipients such as cellulose, lactose, magnesium stearate, starch, and butylated hydroxyanisole as a preservative. When taken separately, lovastatin can treat related hyperlipidemia such as reducing plasma total-C, LDL-C, total-C/HDL-C ratio and LDL-C/HDL-C ratio as well as increasing HDL-C, and decreasing to some degree VLDL-C and plasma triglycerides (TG). Mevacor can lower total-C and LDL-C to target levels, and reduce elevated total-C and LDL-C levels in patients with primary hypercholesterolemia (Types IIa and IIb). Single daily doses given in the evening can be more effective than the same dose given in the morning, perhaps because cholesterol is synthesized mainly at night. A recommended starting dose of Mevacor is preferably given with a meal. 20 mg once a day can be given with the evening meal. Storage between 5-30°C (41-86°F) is preferred.

Fluvastatin (also known as fluvastatin sodium), a synthetic HMG-CoA reductase inhibitor, is a white to pale yellow, hygroscopic powder soluble in water, ethanol and methanol. When formulated for oral administration as Lescol TM, capsules can contain 20 to 40 mg of fluvastatin together with pharmaceutically acceptable excipients such as gelatin, magnesium stearate, microcrystalline cellulose, pregelatinized starch, red iron oxide, sodium lauryl sulfate, talc, titanium dioxide, yellow iron oxide and other ingredients. Fluvastatin sodium reduces Total-C, LDL-C, and apolipoprotein B, and reduces triglycerides (TG) to some extent while producing an increase in HDL-C of variable magnitude. Following oral administration, fluvastatin is absorbed rapidly and completely with peak concentrations reached in less than 1 hour. Administration with food reduces the rate but not the extent of absorption. Fluvastatin sodium is indicated as an adjunct to diet in the treatment of elevated total cholesterol (Total-C), LDL-C, TG and Apo B levels in patients with primary hypercholesterolemia and mixed dyslipidemia (Frederickson Type IIa and IIb). It is also recommended for slowing the progression of coronary atherosclerosis in patients with coronary heart disease as part of a treatment strategy to lower total and LDL cholesterol to target levels.

Atorvastatin (or Atorvastatin calcium 2:1) is a white to off-white crystalline trihydrate powder that is insoluble in aqueous solutions of pH 4 and below, and is very slightly soluble in distilled water, pH 7.4 phosphate buffer, and acetonitrile, slightly soluble in ethanol, and freely soluble in methanol. When formulated for oral administration as Lipitor TM, tablets can contain 10 to 80 mg of atorvastatin as well as pharmaceutically acceptable excipients such as calcium carbonate, USP; candelilla wax, FCC; croscarmellose sodium, NF; hydroxypropyl cellulose, NF; lactose monohydrate, NF; magnesium stearate, NF; microcrystalline cellulose, NF; Opadry White YS-1-7040 (hydroxypropylmethylcellulose, polyethylene glycol, talc, titanium dioxide): polysorbate 80, NF; and simethicone emulsion. Atorvastatin can reduce total-C, LDL-C, and apo B in patients with homozygous and heterozygous familial hypercholesterolemia, nonfamilial forms of hypercholesterolemia, and mixed dyslipidemia. Atorvastatin can also reduce VLDL-C and TG and produces variable increases in HDL-C and apolipoprotein A-1. Atorvastatin can reduce total-C, LDL-C, VLDL-C, apo B, TG, and non-HDL-C, and can increase HDL-C in patients with isolated hypertriglyceridemia. Atorvastatin can reduce intermediate density lipoprotein cholesterol (IDL-C) in patients with dysbetalipoproteinemia. Food decreases the rate and extent of drug absorption as assessed by Cₘₐₓ and AUC, but LDL-C reduction is similar whether atorvastatin is given with or without food. Atorvastatin can be administered as a single dose at any time of the day, with or without food. Atorvastatin can reduce total-C, LDL-C, VLDL-C, apo B, and TG, and can increase HDL-C in patients with hypercholesterolemia and mixed dyslipidemia.

Simvastatin is a white to off-white, nonhygroscopic, crystalline powder that is practically insoluble in water, and freely soluble in chloroform, methanol and ethanol. Simvastatin is derived synthetically from a fermentation product of Aspergillus terreus. After oral ingestion, simvastatin, which is an inactive lactone, is hydrolysed to the corresponding (beta)-hydroxyacid form, which is an inhibitor of 3-hydroxy-3-methyl-glutaryl-coenzyme A (HMG-CoA) reductase. When formulated as Zocor TM for oral administration, tablets can contain 5 mg to 80 mg of simvastatin as well as pharmaceutically acceptable excipients such as cellulose, hydroxypropyl cellulose, hydroxypropyl methylcellulose, iron oxides, lactose, magnesium stearate, starch, talc, titanium dioxide as well as other ingredients including butylated hydroxyanisole which can be added as a preservative. Simvastatin shows no fed-fasted effect when administered immediately before a low-fat meal. Simvastatin can reduce total-C, LDL-C, total-C/HDL-C ratio, and LDL-C/HDL-C ratio as well as decrease TG and increase HDL-C.

Cerivastatin (or Cerivastatin sodium) is a white to off-white hygroscopic amorphous powder that is soluble in water, methanol, and ethanol, and very slightly soluble in acetone. Cerivastatin sodium is a synthetic, enantiomerically pure competitive inhibitor of the enzyme 3-hydroxy-3-methylglutaryl-coenzyme A (HMG-CoA) reductase that catalyzes the conversion of HMG-CoA to mevalonate in an early and rate-limiting step in the biosynthesis of cholesterol. The inhibition of cholesterol biosynthesis reduces the level of cholesterol in hepatic cells, which stimulates the synthesis of LDL receptors and increases the uptake of cellular LDL particles. This can lead to a reduction in plasma cholesterol concentration. When formulated as Baycol TM, cerivastatin sodium tablets can contain 0.2 to 0.8 mg of cerivastatin sodium for oral administration and can be taken with or without food. Other tablet ingredients can include pharmaceutically acceptable excipients such as mannitol, magnesium stearate, sodium hydroxide, crospovidone, povidone, iron oxide yellow, methylhydroxypropylcellulose, polyethylene glycol, and titanium dioxide. In patients with hypercholesterolemia, cerivastatin sodium can produce reduced levels of plasma total cholesterol, LDL-C, and apolipoprotein B, VLDL-C and plasma triglycerides and increases plasma HDL-C and apolipoprotein A-1. Cerivastatin systemic exposure (area under the curve, AUC) and Cₘₐₓ are not sensitive to a food effect, but once daily doses of 0.2 mg can be more efficacious than twice daily doses of 0.1 mg. Cerivastatin sodium can be effective as an adjunct to diet to reduce elevated Total-C, LDL-C, apo B, and TG and to increase HDL-C levels in patients with primary hypercholesterolemia and mixed dyslipidemia (Fredrickson Types IIa and IIb) when the response to dietary restriction of saturated fat and cholesterol and other non-pharmacological measures alone is inadequate.

Pravastatin (or pravastatin sodium) is a white to off-white, fine or crystalline powder. It is a relatively polar hydrophilic compound with a partition coefficient (octanol/water) of 0.59 at a pH of 7.0. It is soluble in methanol and water (>300 mg/mL), slightly soluble in isopropanol, and practically insoluble in acetone, acetonitrile, chloroform, and ether. When formulated as Pravachol TM for oral administration, tablets can contain 10 to 40 mg of pravastatin. Inactive ingredients can include pharmaceutically acceptable excipients such as croscarmellose sodium, lactose, magnesium oxide, magnesium stearate, microcrystalline cellulose, and povidone. A 10 mg tablet can also contain Red Ferric Oxide, a 20 mg tablet can also contain Yellow Ferric Oxide, and a 40 mg tablet can also contain Green Lake Blend (mixture of D&C Yellow No. 10-Aluminum Lake and FD&C Blue No. 1-Aluminum Lake).

Itavastatin is an inhibitor of HMG-CoA reductase and can be dosed in tablets containing from about 1 mg to about 20 mg, preferably from about 2 mg to about 10 mg.

Rosuvastatin is an inhibitor of HMG-CoA reductase and can be dosed in tablets containing from about 4 or 5 mg to about 10 or 20 mg, with reported doses of up to about 80 mg per day when formulated as Crestor TM.

Preferred statins in this invention are those useful for oral administration. According to the invention, the statin can be administered in the form of one of its pharmaceutically acceptable salts. Such salts, for example, can be formed between a negatively charged substituent in a compound (e.g. carboxylate) with a cation. Suitable cations include, but are not limited to, sodium ion, potassium ion, magnesium ion, calcium ion, and an ammonium cation such as tetramethylammonium ion. Among the salts, sodium and calcium are more particularly preferred.

If in the composition used in the present invention, lovastatin is used, the amount to be administered is between 10 to 40 mg, and more preferably 20 mg per day. If fluvastatin is used, the amount is between 20 to 40 mg per day. If atorvastatin is used, the amount is between 10 to 80 mg, and more preferably 10 to 40 mg per day. If simvastatin is used, the amount is between 5 to 50 mg, more preferably 5 to 20 mg per day. If cerivastatin is used, the amount is between 0.1 to 0.8 mg, more preferably 0.1 to 0.3 mg per day. If pravastatin is used, the amount is between 10 to 40 mg, more preferably 20 mg per day. If itavastatin is used, the amount is between 1 to 20 mg, more preferably from 2 to 20 mg per day. If rosuvastatin is used, the amount is between 4 to 80 mg, more preferably 10 to 20 mg per day.

The amount of statin used in the invention is the amount mentioned before with respect to each specific statin. This amount can be chosen between 0.1 mg to 100 mg depending on the specific statin used.

In the invention, the metformin is to be administered in the form of one of its pharmaceutically acceptable salts, such as the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octanoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate or phosphate.

Among these salts, the hydrochloride, fumarate, embonate and chlorophenoxyacetate are more particularly preferred.

The pharmaceutically acceptable salts of metformin are obtained in a manner, which is known per se, by the action of metformin on the corresponding acid.

A once a day amount of metformin is generally from 200 mg to 2000 mg. The most common composition to be used contains from 500 mg and 850 mg of metformin and is to be taken twice or three times a day.

Thus, if the compositions used in the invention contain 500 or 850 mg and are to be taken more than once a day, then the amount of statin will be adjusted consequently.

The amount of metformin and the amount of statin together provide a dosage or amount of the combination that is sufficient to constitute an effective amount of the combination. The effective amount can be a glycaemic or lipidaemic disorder or disease suppressing treatment or an amount sufficient to effect prevention.

As used herein, an "effective amount" means the dose or effective amount to be administered to a patient. The dose or effective amount to be administered to a patient and the frequency of administration to the subject can be readily determined by one of ordinary skill in the art by the use of known techniques and by observing results obtained under analogous circumstances. In determining the effective amount or dose, a number of factors are considered by the diagnostician attending to the case, including but not limited to, the potency and duration of action of the compounds used, the nature and severity of the illness to be treated, as well as the sex, age weight, general health and individual responsiveness of the patient to be treated, and other relevant circumstances.

The compositions as used in the invention contain therapeutically effective amounts of the various active ingredients. The ratio of the respective amounts of statin, or one of its pharmaceutically acceptable salts, and of metformin, or one of its pharmaceutically acceptable salts thus varies in consequence. This ratio of statin, or its pharmaceutically acceptable salt, to metformin, or its pharmaceutically acceptable salt, can vary from 1:2 to 1:20000. It is preferred that the weight ratio of the amount of statin to the amount of metformin that is administered to the subject is within a range of from about 1:2 to about 1:2000, preferably from about 1:4 to about 1:2000 and more preferably from about 1:5 to about 1:2000. The ratio of statin to metformin in the composition of the invention will vary if the present pharmaceutical composition is to be taken more than once a day, or if it is to be only taken once a day.

The expression "therapeutically effective" indicates the capability of an agent to prevent, or reduce the severity of, the disorder being treated, while avoiding adverse side effects typically associated with alternative therapies. The expression "therapeutically effective" is to be understood to be equivalent to the expression "effective for the treatment, prevention, or inhibition", and both are intended to qualify the amount of each agent for use in the combination therapy, which will achieve the goal of improvement in the severity of non insulin dependent diabetes.

The metformin amount is that sufficient to constitute an effective amount of the combination. Preferably, such amount would be sufficient to provide a therapeutically effective amount of the combination. The therapeutically effective amount can also be described herein as a hyperglycaemic treatment or prevention effective amount of the combination.

The relative amounts of each component in the therapeutic composition may be varied and may be as described just above. The metformin and statin that are described above can be provided in the therapeutic composition so that the preferred amounts of each of the components are supplied by a single dosage, a single injection or a single capsule for example, or, by up to two, or more, single dosage forms.

When the combination to be used is supplied along with a pharmaceutically acceptable carrier, a pharmaceutical composition is formed. The pharmaceutical composition to be used according to the present invention is directed to a composition suitable for the prevention or treatment of hyperglycaemia in non insulin dependent diabetes subjects. Such a pharmaceutical composition comprises a pharmaceutically acceptable carrier, a statin and metformin.

Pharmaceutically acceptable carriers include, but are not limited to, physiological saline, Ringer's, phosphate solution or buffer, buffered saline, and other carriers known in the art. Pharmaceutical compositions to be used may also include stabilizers, anti-oxidants, colorants, and diluents, or any pharmaceutical excipients.

The combination to be used in the present invention is useful for, but not limited to, the prevention, inhibition and treatment of hyperglycaemia in non insulin dependent diabetes subjects.

As described above, an embodiment of the present invention comprises the use of a pharmaceutical composition, comprising a therapeutically-effective amount of a combination of metformin and a statin in association with at least one pharmaceutically-acceptable carrier, adjuvant or diluent and, if desired, other active ingredients.

The expressions "combination therapy" and "administration with" in defining the use of a metformin and a statin are intended to embrace co-administration of these agents in a substantially simultaneous manner, such as in a single capsule or dosage device having a fixed ratio of these active agents or in multiple, separate capsules or dosage devices that can be taken together contemporaneously.

The compositions to be used in the invention are preferably to be administered enterally or parenterally (parenteral administration includes subcutaneous, intramuscular, intradermal, intramammary, intravenous, and other methods of administration known in the art), or better still orally, although the other routes of administration, for instance such as rectal administration, are not excluded.

For preparing oral pharmaceutical compositions from the compounds to be used according to this invention, inert, pharmaceutically acceptable carriers can be either solid or liquid: Solid form preparations include powders, tablets, coated tablets, dragees, troches, lozenges, dispersible granules, capsules, and sachets. Compositions for oral use may be prepared according to any method known in the art of manufacture of pharmaceutical compositions.

A solid carrier can be one or more substances which may also act as diluents, flavouring agents, solubilizers, lubricants, suspending agents, binders, or tablet disintegrating agents; it can also be an encapsulating material.

In powders, the carrier is a finely divided solid, which is in a mixture with the finely divided active ingredient(s). In tablets, the active ingredient(s) is (are) mixed with the carrier having the necessary binding properties in suitable proportions and compacted in the shape and size desired.

Suitable carriers may be, for example, inert diluents, such as magnesium carbonate, calcium stearate, magnesium stearate, talc, lactose, sugar, pectin, dextrin, starch, tragacanth, methyl cellulose, sodium carboxymethyl cellulose, and the like.

The present invention also includes the use of formulation of metformin and statin with encapsulating material as a carrier providing a capsule in which metformin and statin (with or without other carriers) is surrounded by a carrier, which is thus in association with metformin and statin. In a similar manner, sachets are also included. Tablets, powders, sachets, and capsules can be used as solid dosage forms suitable for oral administration.

The tablets may be uncoated or coated by known techniques to delay disintegration and adsorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredients are mixed with inert solid diluent, for example, calcium carbonate, calcium phosphate or kaolin, or as soft gelatin capsules wherein the active ingredients are present as such, or mixed with water or an oil medium, for example, arachid oil, liquid paraffin, or olive oil.

Aqueous suspensions can be produced that contain the active compounds in admixture with excipients suitable for the manufacture of aqueous suspensions. Such excipients are suspending agents, for example, sodium carboxymethylcellulose, methylcellulose, hydroxypropylmethylcellulose, sodium alginate, polyvinylpyrrolidone, gum tragacanth and gum acacia; dispersing or wetting agents which include naturally-occuring phosphatides, for example lecithin, or condensation products of an alkylene oxide with fatty acids, for example polyoxyethylene stearate, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan monooleate.

The aqueous suspensions may also contain one or more preservatives, for example, ethyl or n-propyl p-hydroxybenzoate, one or more coloring agents, one or more flavoring agents, one or more sweetening agents.

Oily suspensions may be formulated by suspending the active compounds in an omega-3 fatty acid, a vegetable oil, for example arachid oil, olive oil, sesame oil or coconut oil, or in a mineral oil such as liquid paraffin. The oily suspensions may contain a thickening agent, for example, beeswax, hard paraffin or cetyl alcohol.

Sweetening agents and flavoring agents may be added to provide a palatable oral preparation. This preparation may be preserved by the addition of an antioxidant such as ascorbic acid.

Dispersible powders and granules suitable for preparation of an aqueous suspension by the addition of water provide the active compounds in admixture with a dispersing or wetting agent, a suspending agent and one or more preservatives. Suitable dispersing or wetting agents and suspending agents are exemplified by those already mentioned above. Additional excipients, for example sweetening, flavoring and coloring agents, may also be present.

Syrups and elixirs containing the novel combination may be formulated with sweetening agents. Such formulations may also contain a demulcent, a preservative and flavoring and coloring agents.

Formulations developed for metformin can be used for the pharmaceutical composition to be used according to the invention containing metformin and a statin. Such formulations of the metformin are described in the following patents: Gastric retentive (WO 9907342), controlled release metformin composition (WO 0236100), controlled release with unitary core (WO 9947125), treatment with 400 mg or below of metformin (US 6,100,300), novel salts of metformin (WO 9929314), biphasic controlled release delivery system (WO 9947128), metformin preparation (WO 9608243), gastric retentive (WO 9855107), controlled release (WO 0103964 and WO 0239984), metformin tablet (WO 03004009), sustained release composition (WO 02067905), controlled release composition (WO 0211701), gastroretentive (WO 0006129), solid carriers for improved delivery (WO 0137808), coating for sustained release composition (WO 02085335), modified release composition (WO 03002151), liquid formulation of metformin (WO 0247607), controlled release device (WO 02094227), metformin quick release tablet (JP 2002326927).

Among those formulations, metformin once-a-day formulations are preferred.

Liquid form preparations include solutions, suspensions and emulsions suitable for oral administration. Aqueous solutions for oral administration can be prepared by dissolving the active ingredients in water and adding suitable flavorants, colouring agents, stabilizers, and thickening agents as desired. Ethanol, propylene glycol and other pharmaceutically acceptable non-aqueous solvents may be added to improve the solubility of the active ingredients. Aqueous suspensions for oral use can be made by dispersing the finely divided active compounds in water together with a viscous material such as natural or synthetic gums, resins, methyl cellulose, sodium carboxymethyl cellulose, and other suspending agents known in the pharmaceutical formulation art.

Preferably, the used pharmaceutical preparation is in unit dosage form. In such form, the preparation is divided into unit doses containing appropriate quantities of the active ingredients. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

The subject combinations can also be administered parenterally either subcutaneously, or intravenously, or intramuscularly, or intrasternally, or by infusion techniques, in the form of sterile injectable aqueous or olagenous suspensions. Such suspensions may be formulated according to the known art using suitable dispersing, wetting and suspending agents mentioned above, or other acceptable agents. The sterile injectable preparation may also be a sterile injectable solution or suspension in a non-toxic parenterally-acceptable diluent or solvent, for example as a solution in 1,3-butanediol. Among the acceptable vehicles and solvent that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. In addition, n-3 polyunsaturated fatty acids may find use in the preparation of injectables.

The subject combination can also be administered by inhalation, in the form of aerosols or solutions for nebulizers, or rectally in the form of suppositories prepared by mixing the active ingredients with a suitable non irritating excipient, which is solid at ordinary temperature but liquid at rectal temperature and will therefore melt in the rectum to release the drug. Such materials are cocoa butter and polyethylene glycols.

Preferably, the subject composition to be used is a controlled release composition.

Daily dosages can vary within wide limits and will be adjusted to the individual requirements in each particular case. In general, for administration to adults, an appropriate daily dosage has been described above, although the limits that were identified as being preferred may be exceeded if expedient. The daily dosage can be administered as a single dosage or divided dosages.

The present invention further comprises kits that are suitable for use in performing the treatment described above. In one embodiment, the kit contains a first dosage form comprising metformin in one or more of the forms identified above and a second dosage form comprising one or more of the statin identified above, for a simultaneous administration, in quantities sufficient to carry out the use of the present invention.

The following examples describe embodiments of the invention. Other embodiments within the scope of the claims herein will be apparent to one skilled in the art from consideration of the specification or practice of the invention as disclosed herein. It is intended that the specification, together with the examples, be considered to be exemplary only, with the scope and spirit of the invention being indicated by the claims, which follow the examples.

### Example 1

The synergism of action was proven using an animal model. Zucker obese (fa/fa) rats were used as a model of non-insulin-dependent diabetes (NIDD). The action of lovastatin alone, of metformin alone and of the combination lovastatin + metformin was evaluated in terms of triglycerides, total cholesterol, High Density Lipoprotein-C (HDL C), glucose and insulin. The rats were given the treatment for five consecutive days. Blood samples were collected three days before and five days after the beginning of the treatments in order to measure triglycerides, total cholesterol, HDL C, glucose and insulin levels. The procedure followed was:
Four groups of 8 rats were formed:
   - a vehicle group,
   - a group who received a dose of 1 mg/kg/day of lovastatin per os,
   - a group who received a dose of 50 mg/kg twice a day of metformin per os,
   - a group who received a dose of 1 mg/kg/day of lovastatin + 50 mg/kg twice a day of metformin per os.

Statistical analysis consists in one-way analysis of variance followed by multiple comparisons versus the vehicle group (Dunnett's t test) to evaluate the significance results obtained; values are expressed as mean ± S.E.M. A difference will be considered significant (*) for p<0.05. Results are expressed in millimol per liter (mM) or nanomol per liter (nM).

The results are reported in table 1 below:

**Table 1. Effects of lovastatin alone, metformin alone and lovastatin+metformin on serum biomarkers in Zucker obese (fa/fa) rats dosed per os for five days.**

| Treatment mg/kg | Triglycerides (mM) | | Total Cholesterol (mM) | | HDL C (mM) | | Glucose (mM) | | Insulin (nM) | |
|---|---|---|---|---|---|---|---|---|---|---|
| | T0 | T5 | T0 | T5 | T0 | T5 | T0 | T5 | T0 | T5 |
| Vehicle | 4.22 | 4.79 | 2.42 | 2.53 | 1.95 | 2.12 | 8.44 | **8.28** | 0.67 | 0.49 |
| | *0.35* | *0.32* | *0.06* | *0.06* | *0.04* | *0.06* | *0.62* | *0.36* | *0.15* | *0.05* |
| Lovastatin | 4.18 | 4.79 | 2.38 | 2.54 | 2.00 | 2.16 | 7.88 | **8.21** | 0.35 | 0.39 |
| 1 mg/kg, p.o. | *0.41* | *0.48* | *0.11* | *0.10* | *0.08* | *0.08* | *0.44* | *0.26* | *0.09* | *0.07* |
| Metformin | 4.08 | 5.17 | 2.55 | 2.72 | 2.08 | 2.25 | 8.27 | **7.73** | 0.34 | 0.38 |
| 50 mg/kg, p.o. | *0.32* | *0.42* | *0.11* | *0.11* | *0.08* | *0.08* | *0.69* | *0.31* | *0.03* | *0.02* |
| Lovastatin+Metformin | 4.10 | 4.40 | 2.40 | 2.43 | 1.96 | 2.07 | 7.69 | **7.00*** | 0.29 | 0.36 |
| 1+50 mg/kg, p.o. | *0.33* | *0.30* | *0.10* | *0.06* | *0.08* | *0.06* | *0.54* | *0.23* | *0.05* | *0.05* |

The results obtained show the synergism of action of lovastatin and metformin on glycaemia.

Metformin used alone leads to a glycaemia of 7.73 mM and lovastatin alone leads to a glycaemia of 8.21 mM. The combination of metformin and lovastatin leads to a glycaemia of 7.00 mM.

### Example 2

The action of simvastatin alone, of metformin alone and of the combination simvastatin + metformin was evaluated in terms of triglycerides, total cholesterol, High Density Lipoprotein-C (HDL C), glucose and insulin.

Male 10/11 weeks old ZUCKER fa/fa rats (Charles River, France) were used in the study. They were housed 2 per cage in a temperature (21-24.5°C) and relative humidity (45-65%) controlled room with a 12-h light/dark cycle, with *ad libitum* access to filtered tap-water and standard pelleted laboratory chow (SAFE, France) throughout the study. After acclimatization, they were randomized into 6 groups of 8 according to their triglyceridemia:
- Group 1: vehicle
- Group 2: simvastatin 0.5 mg/kg
- Group 3: simvastatin 1 mg/kg
- Group 4: metformin 50 mg/kg (twice a day)
- Group 5: simvastatin 0.5 mg/kg + metformin 50 mg/kg (twice a day)
- Group 6: simvastatin 1 mg/kg + metformin 50 mg/kg (twice a day).

Rats were given the treatment orally once (simvastatin) or twice a day (metformin) for five consecutive days at constant time. In groups 5 and 6, simvastatin was administered at the time of the second administration of metformin. Blood samples were collected three days before and five days after the beginning of the treatment in order to measure the above-mentioned parameters.

The results are reported in Table 2 below. These results are expressed in millimol per liter (mM) or nanomol per liter (nM).

Values are expressed as mean ± S.E.M. Statistical analysis consists in:
- either one-way analysis of variance (ANOVA) followed by Dunnett's t test; a difference is considered significant if P ≤ 0.05 vs vehicle (*).
- or Student-Newman-Keuls test; a difference is considered significant if P ≤ 0.05 vs simvastatin 0.5 mg/kg ($), or P < 0.05 vs metformin 50 mg/kg (†).

**Table 2. Effects of simvastatin alone, metformin alone and simvastatin + metformin on serum biomarkers in Zucker obese (fa/fa) rats dosed per os for 5 days**

| Treatment mg/kg | Triglycerides (mM) | | Total Cholesterol (mM) | | HDL C (mM) | | Glucose (mM) | | Insulin (nM) | |
|---|---|---|---|---|---|---|---|---|---|---|
| | T0 | T5 | T0 | T5 | T0 | T5 | T0 | T5 | T0 | T5 |
| Vehicle | 4.21 | 5.01 | 2.49 | 2.50 | 2.34 | 2.37 | 6.67 | **9.84** | 0.47 | 0.61 |
| | *0.33* | *0.41* | *0.13* | *0.12* | *0.10* | *0.10* | *0.32* | *0.65* | *0.03* | *0.16* |
| Simvastatin | 4.61 | 5.23 | 2.46 | 2.46 | 2.32 | 2.32 | 6.63 | **9.44** | 0.55 | 0.76 |
| 0.5 mg/kg | *0.20* | *0.39* | *0.11* | *0.13* | *0.10* | *0.12* | *0.18* | *0.68* | *0.10* | *0.13* |
| Simvastatin | 4.67 | 5.89 | 2.48 | 2.60 | 2.34 | 2.43 | 7.08 | **8.35** | 0.63 | 0.67 |
| 1 mg/kg | *0.42* | *0.47* | *0.11* | *0.14* | *0.12* | *0.12* | *0.48* | *0.29* | *0.11* | *0.09* |
| Metformin | 4.18 | 5.12 | 2.47 | 2.32 | 2.30 | 2.16 | 6.69 | **9.23** | 0.63 | 0.71 |
| 50 mg/kg | *0.28* | *0.37* | *0.14* | *0.14* | *0.12* | *0.11* | *0.21* | *0.36* | *0.10* | *0.15* |
| Simvastatin 0.5 mg/kg | 5.04 | 6.24 | 2.58 | 2.58 | 2.40 | 2.43 | 7.13 | **7.14** | 0.59 | 0.49 |
| + Metformin 50 mg/kg | *0.44* | *0.55* | *0.11* | *0.05* | *0.08* | *0.04* | *0.38* | *0.15* */†/$ | *0.11* | *0.04* |
| Simvastatin 1 mg/kg | 4.35 | 5.32 | 2.35 | 2.37 | 2.22 | 2.29 | 6.44 | 7.46 | 0.48 | 0.57 |
| + Metformin 50 mg/kg | *0.34* | *0.58* | *0.17* | *0.17* | *0.13* | *0.14* | *0.13* | *0.29* */† | *0.06* | *0.16* |

The results obtained show the synergism of action of simvastatin and metformin on glycaemia.

Metformin used alone leads to a glycaemia of 9.23 mM and simvastatin alone leads to a glycaemia of 9.44 mM (0.5 mg/kg) or 8.35 mM (1 mg/kg). The combination of metformin and simvastatin leads to a glycaemia of 7.14 mM (0.5 mg/kg simvastatin) or 7.46 mM (1 mg/kg simvastatin).

### Example 3

The action of simvastatin alone, of metformin alone and of the combination simvastatin + metformin was evaluated in terms of triglycerides, total cholesterol, non esterified fatty acids (NEFA), glucose and insulin.

Male 12 weeks old C57BL/Ks J Rj-db (db/db) mice (Janvier, France), weighing in the target range of 30 to 50 g, were used in the study. They were housed 5 per cage in a temperature (19.5-24.5°C) and relative humidity (45-65%) controlled room with a 12-h light/dark cycle, with *ad libitum* access to filtered tap-water and irradiated pelleted laboratory chow (ref. A04, SAFE, France) throughout the study. After acclimatization, they were randomized into groups of 10 according to homogeneous glycaemia:
- Group 1 : Vehicle
- Group 2 : Metformin 150 mg/kg
- Group 3 : Simvastatin 30 mg/kg
- Group 4 : Metformin 150 mg/kg + Simvastatin 30 mg/kg
- Group 5 : Simvastatin 300 mg/kg
- Group 6 : Metformin 150 mg/kg + Simvastatin 300 mg/kg.

The mice were given the treatment orally once daily for 5 consecutive days at constant time. Blood samples were collected three days before and five days after the beginning of the treatment in order to measure the above-mentioned parameters.

The results are reported in Table 3 below. These results are expressed in millimol per liter (mM) or nanomol per liter (nM).

Values are expressed as mean ± S.E.M. Statistical analysis consists in one-way analysis of variance (ANOVA) followed by a Student-Newman-Keuls test; a difference is considered significant if P ≤ 0.05 vs vehicle (*) or if P ≤ 0.05 vs simvastatin 30 mg/kg (°).

**Table 3. Effects of simvastatin alone, metformin alone and simvastatin + metformin on serum biomarkers in male db/db mice dosed per os for 5 days**

| Treatment mg/kg | Triglycerides (mM) | | Total Cholesterol (mM) | | NEFA (mM) | | Glucose (mM) | | Insulin (nM) | |
|---|---|---|---|---|---|---|---|---|---|---|
| | T0 | T5 | T0 | T5 | T0 | T5 | T0 | T5 | T0 | T5 |
| Vehicle | 1.39 | 1.80 | 3.12 | 3.42 | 0.632 | 0.791 | 36.90 | **31.58** | 4.91 | 2.91 |
| | *0.10* | *0.16* | *0. 06* | *3.42* | *0.050* | *0.056* | *2.18* | *1.32* | *0.62* | *0. 67* |
| Metformin | 1.30 | 1.34 | 3.07 | 3.63 | 0.654 | 0.798 | 37.00 | **23.03** | 4.60 | 2.82 |
| 150 mg/kg | *0.18* | *0.12* | *0.07* | *0.11* | *0.048* | *0.051* | *2.03* | *2.02* * | *0.71* | *0.51* |
| Simvastatin | 1.34 | 1.49 | 2.96 | 3.33 | 0.646 | 0.897 | 36.27 | **25.95** | 4.60 | 2.74 |
| 30 mg/kg | *0.10* | *0.09* | *0.12* | *0.16* | *0.038* | *0.039* | *2.00* | *1.83* * | *0.72* | *0.35* |
| Metformin 150 mg/kg | 1.33 | 1.13 | 3.05 | 3.17 | 0.622 | 0.721 | 36.73 | **22.97** | 5.44 | 3.25 |
| + Simvastatin 30 mg/kg (n=9) | *0.16* | *0.09* | *0.06* | *0.07* | *0.065* | *0.044* | *1.99* | *2.34* * | *0.72* | *0.45* |
| Simvastatin | *1.44* | *0.78* | *2.95* | *2.87* | *0.639* | *0.785* | *36.91* | **21.18** | *4.86* | *2.37* |
| 300 mg/kg | *0.16* | *0.06* | *0.20* | *0.22* | *0.054* | *0.055* | *2.13* | *2.13* * | *0.98* | *0.61* |
| Metformin 150 mg/kg | 1.11 | 0.60 | 3.17 | 2.61 | 0.623 | 0.617 | 36.91 | **17.12** | 6.85 | 2.38 |
| + Simvastatin 300 mg/kg | *0.17* | *0.06* | *0.07* | *0.08* | *0.027* | *0.031* | *2.22* | *2.27* */° | *0.95* | *0.37* |

The results obtained show that the combined action of simvastatin and metformin makes it possible to control or lower glycaemia.

Metformin used alone leads to a glycaemia of 23.03 mM and simvastatin alone leads to a glycaemia of 25.95 mM (30 mg/kg) or 21.18 mM (300 mg/kg). The combination of metformin and simvastatin leads to a glycaemia of 22.97 mM (30 mg/kg simvastatin) or 17.12 mM (300 mg/kg simvastatin).

## Claims

1. Use of metformin, a statin and one or more pharmaceutically acceptable excipients, for the manufacture of a pharmaceutical composition for controlling or decreasing glycaemia in non insulin dependent diabetes subjects.

2. The use according to claim 1, wherein the statin is selected from the group consisting of lovastatin, fluvastatin, atorvastatin, simvastatin, pravastatin, itavastatin and rosuvastatin.

3. The use according to claim 1 or 2, wherein metformin is in the form of a salt selected from the group consisting of the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octanoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate and phosphate.

4. The use according to any of claims 1 to 3, wherein metformin is in the form of a salt selected from the group consisting of the hydrochloride, fumarate, embonate, and chlorophenoxyacetate.

5. The use according to any of claims 1 to 4, wherein the statin is in the form of a salt selected from the group consisting of the sodium ion, potassium ion, magnesium ion, calcium ion, and an ammonium cation such as tetramethylammonium ion.

6. The use according to any of claims 1 to 5, wherein said composition contains from 0.1 to 100 mg of a statin.

7. The use according to any of claims 1 to 6, wherein said composition contains from 200 to 2000 mg of metformin.

8. The use according to any of claims 1 to 7, wherein the weight ratio of statin to metformin is in the range of about 1:2 to about 1:20000.

9. The use according to any of claims 1 to 8, wherein said composition is in the form of powders, tablets, coated tablets, dragees, troches, lozenges, dispersible granules, capsules or sachets.

10. The use according to any of claims 1 to 8, wherein said composition is in the form of a solution, a suspension or an emulsion.

11. The use according to any of claims 1 to 10, wherein the pharmaceutical composition is a controlled-release composition.

12. The use according to any of claims 1 to 11, wherein the pharmaceutical composition is administered orally.

13. Use of metformin and a statin in the manufacture of a kit comprising metformin, or one of its pharmaceutically acceptable salts, and a statin, or one of its pharmaceutically acceptable salts, for the simultaneous co-administration of metformin and the statin for controlling or decreasing glycaemia in non insulin dependent diabetes subjects.

14. Combination of metformin and a statin for use in controlling or decreasing glycaemia in non insulin dependent diabetes subjects.

## Patentansprüche

1. Verwendung von Metformin, eines Statins und eines oder mehrerer pharmazeutisch zulässiger Arzneiträger zur Herstellung einer pharmazeutischen Zusammensetzung zur Glycaemiesteuerung oder -reduzierung bei nicht insulinabhängigen Diabetespatienten.

2. Verwendung nach Anspruch 1, wobei das Statin aus einer Gruppe umfassend Lovastatin, Fluvastatin, Atrovastatin, Simvastatin, Pravastatin, Itavastatin und Rosuvastatin ausgewählt ist.

3. Verwendung nach Anspruch 1 oder 2, wobei Metformin in Form eines Salzes vorliegt, das aus einer Gruppe umfassend das Hydrochlorid, Acetat, Benzoat, Citrat, Fumarat, Embonat, Chlorphenoxyacetat, Glycolat, Palmoat, Aspartat, Methansulfonat, Maleat, Parachlorphenoxyisobutyrat, Format, Lactat, Succinat, Sulfat, Tartrat, Cyclohexancarboxylat, Hexanat, Octanat, Decanat, Hexadecanat, Octodecanat, Benzolsulfonat, Trimethoxybenzoat, Paratoluensulfonat, Adamantancarboxylat, Glycoxylat, Glutamat, Pyrrolidoncarboxylat, Naphtalensulfonat, 1-Glucosephosphat, Nitrat, Sulfit, Dithionat und Phosphat ausgewählt ist.

4. Verwendung nach einem der Ansprüche 1 bis 3, wobei Metformin in Form eines Salzes vorliegt, das aus einer Gruppe umfassend Hydrochlorid, Fumarat, Embonat und Chlorphenoxyacetat ausgewählt ist.

5. Verwendung nach einem der Ansprüche 1 bis 4, wobei das Statin in Form eines Salzes vorliegt, das aus einer Gruppe umfassend das Natriumion, Kaliumion, Magnesiumion, Calciumion und ein Ammoniumkation wie Tetramethylammoniumion ausgewählt ist.

6. Verwendung nach einem der Ansprüche 1 bis 5, wobei die Zusammensetzung zwischen 0,1 und 100 mg Statin enthält.

7. Verwendung nach einem der Ansprüche 1 bis 6, wobei die Zusammensetzung zwischen 200 und 2.000 mg Metformin enthält.

8. Verwendung nach einem der Ansprüche 1 bis 7, wobei das Gewichtsverhältnis von Statin zu Metformin im Bereich von etwa 1:2 bis etwa 1:20.000 liegt.

9. Verwendung nach einem der Ansprüche 1 bis 8, wobei die Zusammensetzung in Form von Pulvern, Tabletten, überzogenen Tabletten, Dragees, Pastillen, Lutschtabletten, dispergierbaren Granulaten, Kapseln oder Päckchen vorliegt.

10. Verwendung nach einem der Ansprüche 1 bis 8, wobei die Zusammensetzung in Form einer Lösung, einer Suspension oder einer Emulsion vorliegt.

11. Verwendung nach einem der Ansprüche 1 bis 10, wobei die pharmazeutische Zusammensetzung eine Retardzusammensetzung ist.

12. Verwendung nach einem der Ansprüche 1 bis 11, wobei die pharmazeutische Zusammensetzung oral verabreicht wird.

13. Verwendung von Metformin und eines Statins bei der Herstellung eines Kits umfassend Metformin oder eines seiner pharmazeutisch zulässigen Salze und ein Statin oder eines seiner pharmazeutisch zulässigen Salze für die zeitgleiche Abgabe von Metformin und dem Statin zur Glycaemiesteuerung oder -reduzierung bei nicht insulinabhängigen Diabetespatienten.

14. Zusammensetzung von Metformin und einem Statin zur Verwendung bei der Glycaemiesteuerung oder -reduzierung bei nicht insulinabhängigen Diabetespatienten.

## Revendications

1. Utilisation de metformine, d'une statine et d'un ou plusieurs excipients pharmaceutiquement acceptables pour la fabrication d'une composition pharmaceutique destinée à contrôler ou à abaisser la glycémie chez les sujets diabétiques non insulinodépendants.

2. Utilisation selon la revendication 1, où la statine est sélectionnée dans le groupe constitué de la lovastatine, la fluvastatine, l'atorvastatine, la simvastatine, la pravastatine, l'itavastatine et la rosuvastatine.

3. Utilisation selon la revendication 1 ou 2, où la metformine se présente sous la forme d'un sel sélectionné dans le groupe constitué du chlorhydrate, de l'acétate, du benzoate, du citrate, du fumarate, de l'embonate, du chlorophénoxyacétate, du glycolate, du palmoate, de l'aspartate, du méthanesulfonate, du maléate, du parachlorophénoxyisobutyrate, du formate, du lactate, du succinate, du sulfate, du tartrate, du cyclohexanecarboxylate, de l'hexanoate, de l'octanoate, du décanoate, de l'hexadécanoate, de l'octodécanoate, du benzènesulphonate, du triméthoxybenzoate, du paratoluènesulfonate, de l'adamantanecarboxylate, du glycoxylate, du glutamate, du pyrrolidonecarboxylate, du naphtalènesulfonate, du 1-glucosephosphate, du nitrate, du sulfite, du dithionate et du phosphate.

4. Utilisation selon l'une quelconque des revendications 1 à 3, où la metformine se présente sous la forme d'un sel sélectionné dans le groupe constitué du chlorhydrate, du fumarate, de l'embonate, et du chlorophénoxyacétate.

5. Utilisation selon l'une quelconque des revendications 1 à 4, où la statine se présente sous la forme d'un sel sélectionné dans le groupe constitué de l'ion sodium, de l'ion potassium, l'ion magnésium, l'ion calcium, et un cation ammonium tel que l'ion tétraméthylammonium.

6. Utilisation selon l'une quelconque des revendications 1 à 5, où ladite composition contient de 0,1 à 100 mg d'une statine.

7. Utilisation selon l'une quelconque des revendications 1 à 6, où ladite composition contient de 200 à 2 000 mg de metformine.

8. Utilisation selon l'une quelconque des revendications 1 à 7, où le rapport pondéral de la statine à la metformine est dans la plage d'environ 1:2 à environ 1:20 000.

9. Utilisation selon l'une quelconque des revendications 1 à 8, où ladite composition se présente sous la forme de poudres, comprimés, comprimés enrobés, comprimés dragéifiés, tablettes, pastilles, granules dispersibles, gélules ou sachets.

10. Utilisation selon l'une quelconque des revendications 1 à 8, où ladite composition se présente sous la forme d'une solution, d'une suspension ou d'une émulsion.

11. Utilisation selon l'une quelconque des revendications 1 à 10, où la composition pharmaceutique est une composition à libération contrôlée.

12. Utilisation selon l'une quelconque des revendications 1 à 11, où la composition pharmaceutique est administrée oralement.

13. Utilisation de metformine et d'une statine dans la préparation d'un kit comprenant de la metformine ou un de ses sels pharmaceutiquement acceptables, et une statine ou un de ses sels pharmaceutiquement acceptables, pour la co-administration simultanée de metformine et de la statine pour contrôler ou abaisser la glycémie chez les sujets diabétiques non insulinodépendants.

14. Combinaison de metformine et d'une statine pour utilisation dans le contrôle ou l'abaissement de la glycémie chez les sujets diabétiques non insulinodépendants.
